# EUROPEAN PATENT APPLICATION

(11) **EP 4 830 640 A1**
(43) Date of publication of application: **28.10.2026**
(21) Application number: 25172205.4
(22) Date of filing: 24.04.2025
(51) Int. Cl.: A61B 10/02

(54) **BIOPSY GUN**

(71) Applicant: Xaga Surgical AB, 256 67 Helsingborg (SE)
(72) Inventor: FORSVALL, Andreas, 256 67 HELSINGBORG (SE); UVNÄS, Krister, 247 35 SÖDRA SANDBY (SE); EDBERG, Mats, 241 37 ESLÖV (SE); CARMONIUS, Ola, 214 36 MALMÖ (SE)
(74) Representative: Öberg & Sjöberg AB

(57) **Abstract**

A biopsy arrangement (10) is proposed that comprises a biopsy needle (12) and a biopsy gun (14). The biopsy needle (12) comprises a stylet (16) and a cannula (18), and the biopsy needle (12) has a closed configuration and an open configuration. The biopsy gun (14) comprises a first actuator (40), a second actuator (42), and a travel limiter (44). The first actuator (40) is coupled to the stylet (16) and is arranged to transition the biopsy needle (12) from the closed configuration to the open configuration. The second actuator (42) is coupled to the cannula (18) and arranged to transition the biopsy needle (12) from the open configuration to the closed configuration. The travel limiter (44) is arranged to releasably restrict the second actuator (42) to transition the biopsy needle (12) to the closed configuration at a transition from the open configuration to the closed configuration.

## Description

### Technical field

The proposed technology relates generally to the field of biopsy needles and biopsy guns.

### Background

In commonly used biopsy needles, such as the "Tru-Cut needle", when the biopsy needle transitions from the open configuration to the closed configuration, the head of the stylet can enter the cannula, and the cannula does not impact the head at the closing. There are biopsy needles in which the cannula impacts the head of the stylet when transitioning to the closed configuration, for example the biopsy needles described in WO2018028837A1.

It is advantageous if the biopsy needle transitions rapidly to the closed configuration, since the cutting action improves with the speed of the cannula and the biopsy sample becomes more well defined. However, the impact on the head of the stylet can be hard at a rapid transition, and there is risk for damage on the cannula or the head, and even for the head breaking off the stylet. Thus, there is a need for a biopsy gun that can rapidly transition the cannula in a cutting action and at the same time have a soft transition to the closed configuration. More generally, there is a need for a biopsy gun that lessens the impact on the head. At the same time, there is a need for disposable biopsy arrangements, and it would be advantageous to have a biopsy gun that can be manufactured at a low cost.

### Object

It is an object of the proposed technology to provide a biopsy gun that reduces or limits the impact between the cannula and the stylet of a biopsy needle at a transition to the closed configuration. It is a further object to provide such a biopsy gun with an efficient cutting action between the open configuration and the closed configuration. A further object is to provide a biopsy gun that is simple in function and can be manufactured at low costs.

### Summary

In a first aspect of the proposed technology, a biopsy gun for a biopsy needle comprises a stylet and a cannula, or sheath, and the biopsy needle has a closed configuration, or closed setting, and an open configuration, or open setting. The biopsy gun comprises: a first actuator, a second actuator, and a travel limiter, buffer stop, or abutment. The first actuator is configured to be coupled to, or connected to, the stylet, and the first actuator is configured to transition the biopsy needle from the closed configuration to the open configuration. The second actuator is configured to be coupled to, or connected to, the cannula, and the second actuator is configured to transition the biopsy needle from the open configuration to the closed configuration. The travel limiter may be configured to, or arranged to, releasably restrict, or releasably prevent, the second actuator to transition the biopsy needle to the closed configuration at, or in, a transition from the open configuration to the closed configuration.

In a second aspect of the proposed technology, a biopsy arrangement comprises a biopsy needle and a biopsy gun. The biopsy needle comprises: a stylet and a cannula, and the biopsy needle has a closed configuration, or closed setting, and an open configuration, or open setting. The biopsy gun comprises: a first actuator, a second actuator, and a travel limiter, or buffer stop. The first actuator is coupled to, or connected to, the stylet, and the first actuator is configured to, or arranged to, transition the biopsy needle from the closed configuration to the open configuration. The second actuator is coupled to, or connected to, the cannula, and the second actuator is configured to, or arranged to, transition the biopsy needle from the open configuration to the closed configuration. The travel limiter may be configured to, or arranged to, releasably restrict, releasably prevent, or releasably interrupt, the second actuator to transition the biopsy needle to the closed configuration at, or in, a transition from the open configuration to the closed configuration.

Worded differently, the travel limiter may be configured to, or arranged to, at a transition between the open configuration to the closed configuration, releasably restrict, releasably prevent, or releasably interrupt, the second actuator, or more precisely an action of the second actuator causing the transition of the biopsy needle from the open configuration to the closed configuration. It is understood that this may be between the open configuration and the closed configuration of the biopsy needle.

The travel limiter contributes to reducing or limiting an impact between the cannula and the stylet at a transition from the open configuration to the closed configuration. At the same time, it allows for an efficient cutting action.

It is understood that the stylet and the cannula move relative to one another at a transition between the closed configuration and the open configuration. It is further understood that the first actuator is configured to move the stylet relative to the cannula, and that the second actuator is configured to move the cannula relative to the stylet.

In the biopsy needle, the cannula may impact the stylet in the closed configuration at a transition from the open configuration. Worded differently, the stylet may arrest, or stop, the cannula at a transition from the open configuration to the closed configuration, or it may prevent the cannula from moving forward relative to the stylet in the closed configuration.

The biopsy needle may have an intermediate configuration, or intermediate setting, between the open configuration and the closed configuration. It is understood that the biopsy needle transitions between the closed configuration and the open configuration via the intermediate configuration.

It is specified that the travel limiter may be arranged to releasably restrict the second actuator to transition the biopsy needle to the closed configuration. Worded differently, the travel limiter is arranged to releasably set the biopsy needle in the intermediate configuration, or intermediate setting. It is understood that this is at, or in, a transition from the open configuration to the closed configuration. The cannula may be still, or unmoving, relative to the stylet in the intermediate configuration. The travel limiter may be configured, or arranged, to manually release the biopsy needle from the intermediate configuration. Worded differently, the travel limiter may be manually operated to allow the biopsy needle to transition from the intermediate configuration to the closed configuration.

It is understood that the first actuator is configured to transition the biopsy needle from the closed configuration to the open configuration at an activation of the first actuator. It is further understood that the second actuator is configured to transition the biopsy needle from the open configuration to the closed configuration at an activation of the second actuator.

It is understood that the travel limiter is configured to allow the second actuator to transition the biopsy needle from the intermediate configuration to the closed configuration at an activation, or a release, of the travel limiter.

The first actuator may have a loaded state, or cocked state, and an unloaded state, or uncocked state. Similarly, the second actuator may have a loaded state, or cocked state, and an unloaded state, or uncocked state. The first actuator and the second actuator may be configured to set the biopsy needle in the closed configuration with the first actuator in the loaded state and the second actuator in the loaded state, to set the biopsy needle in the open configuration with the first actuator in the unloaded state and the second actuator in the loaded state, and to set the biopsy needle in the closed configuration with the first actuator in the unloaded state and the second actuator in the unloaded state.

The travel limiter may be arranged to releasably set, or arrest, the second actuator in an intermediate state between the loaded state and the unloaded state of the second actuator. It is understood that in the intermediate state, the second actuator and the travel limiter may jointly set the biopsy needle in the intermediate configuration. It is further understood that the travel limiter is configured to allow the second actuator to transition the biopsy needle from the intermediate configuration to the closed configuration at an activation, or a release, of the travel limiter. It is further understood that the travel limiter is configured to allow the second actuator to transition from the intermediate state to the unloaded state at an activation, or release, of the travel limiter. The cannula may be still, or unmoving, relative to the stylet in the intermediate state of the second actuator.

The first actuator may be a linear actuator. More specifically, the first actuator may have a linear action. Similarly, the second actuator may be a linear actuator. More specifically, the second actuator may have a linear action.

The first actuator may have a first stroke length between the loaded state and the unloaded state of the first actuator. Worded differently, the stylet may move a first stroke length relative to the cannula at a transition between the closed configuration and the open configuration. Similarly, the second actuator may have a second stroke length between the loaded state and the unloaded state of the second actuator. Worded differently, the cannula may move a second stroke length relative to the stylet at a transition between the open configuration and the closed configuration.

It is understood that the first stroke length and the second stroke length may be the same. For example, the first stroke length or the second stroke length may be greater than 3 mm, greater than 5 mm, or greater than 10 mm, and the stroke length may be smaller than 50 mm, smaller than 40 mm, or smaller than 30 mm.

The second actuator may have an intermediate stroke length between the intermediate state and the unloaded state of the second actuator. Worded differently, the cannula may move an intermediate stroke length relative to the stylet at a transition between the intermediate configuration and the closed configuration.

The intermediate stroke length may be greater, or longer, than 2%, 5%, or 10%, of the second stroke length. The intermediate stroke length may be smaller, or shorter, than 20%, or 15%, of the second stroke length.

It is understood that the biopsy gun may further comprise: a support structure, or frame. The support structure may support, or operationally support, the first actuator, the second actuator, and/or the travel limiter. It is understood that the biopsy needle extends from the support structure. It is further understood that the support structure may be elongated, and that the biopsy needle may be aligned with the elongated support structure.

The biopsy gun may further comprise: a trigger arrangement, or trigger assembly. The trigger arrangement may be configured to activate, or release, the travel limiter. It is understood that the trigger assembly may be manually operated. More specifically, the trigger arrangement may be operationally coupled to, or connected to, the first actuator, the second actuator, and the travel limiter. The trigger arrangement may be configured to sequentially activate the first actuator, the second actuator, and the travel limiter. It is understood that the activation is in said order.

The trigger arrangement may comprise a trigger. It is understood that the trigger may be manually operated. The trigger may be a rigid structure. The trigger may have a first position, a second position, and a third position, relative to the support structure. The trigger may activate the first actuator at the first position, the trigger may activate the second actuator at the second position, and the trigger may activate, or release, the travel limiter at the third position. It is understood that this is with the first actuator in the loaded state and the second actuator in the loaded state. It is further understood that the trigger transitions between the first position and the third position via the second position. The trigger may further have a rest position relative to the support structure, and it is understood that the trigger transitions between the rest position and the third position via the first position and the second position. The trigger may move between 1 mm and 4 mm, or between 2 mm and 3 mm, between the first position and the second position. This contributes to a sufficient delay between the activation of the first actuator and the second actuator for the first actuator to come to rest before the second actuator is activated when the trigger is manually operated. The trigger may move between 1 mm and 4 mm, or between 2 mm and 3 mm, between the second position and the third position. This contributes to a sufficient delay between the activation of the second actuator and the travel limiter for the second actuator to come to rest before the travel limiter is activated when the trigger is manually operated.

The trigger arrangement may comprise a return spring arranged to bias the trigger towards the first position, or the rest position. It is understood that the return spring may be biased between, or referenced between, the trigger and the support structure. The return spring may have a spring constant that is great then 0.6 N/mm, or greater than 0.8 N/mm. This contributes to a sufficient delay between the activation of the first actuator and the second actuator for the first actuator to come to rest before the second actuator is activated when the trigger is manually operated. It also contributes to a sufficient delay between the activation of the second actuator and the travel limiter for the second actuator to come to rest before the travel limiter is activated when the trigger is manually operated.

The trigger may be arranged to move, or translate, linearly between the first position, the second position, and the third position. This may also include the rest position. The support structure may form a trigger track, or trigger guide, that restricts the trigger to a linear transition between the first position, the second position, and the third position. This may also include the rest position.

The second position may be located between the first position and the third position. This means that the travel limiter is activated at a forward movement of the trigger relative to the biopsy needle or the support structure, or at a push on the trigger.

The trigger may comprise, or form, a push button. It is understood that the trigger is configured to be manually operated and transitions between the different positions at a pushing on the trigger relative to the support structure. It is understood that the push button is accessible from outside the support structure.

The first actuator may comprise a first spring that biases the first actuator from the loaded state to the unloaded state. It is understood that the biasing of the first spring may be smaller in the unloaded state than in the loaded state. It is further understood that the first spring may bias the first actuator with a spring force that is smaller in the unloaded state than in the loaded state. The spring force may decrease linearly between the loaded state and the unloaded state.

More specifically, the first actuator may comprise a first spring and a first sled, or first hammer, wherein the first sled is configured to be connected to, or attached to, the stylet, or the first sled is connected to, or attached to, the stylet. The first sled has a loaded position, or cocked position, and an unloaded position, or uncocked position, relative to the support structure, and the first spring biases the first sled to transition from the loaded position to the unloaded position. More generally, the first spring biases the biopsy needle to transition from the closed configuration to the open configuration. It is understood that the loaded position is in the loaded state of the first actuator and that the unloaded position is in the unloaded state of the first actuator. It is understood that the first spring may be biased, or referenced, between the first sled and the support structure.

The first sled may be arranged to move, or translate, linearly between the loaded position and the unloaded position. The support structure and/or the trigger may form a first track, or first guide, that restricts the first sled to a linear transition between the loaded position and the unloaded position.

It is understood that the first spring may bias the first sled against the support structure in the unloaded state of the first actuator, or in the unloaded position of the first sled.

The biopsy gun, or the first actuator, may comprise a first catch arrangement, or first sear mechanism, arranged to releasably lock, or arrest, the first actuator in the loaded state, or more precisely to releasably lock, or arrest, the first sled in the loaded position. The trigger may be arranged to cooperate with the first catch arrangement and to activate the first actuator, to unlock, or release, the first actuator from the loaded state, or more precisely to unlock, or release, the first sled from the loaded position.

The first catch arrangement may comprise a first cantilever and a first seat, or first post. The first cantilever may be a leaf spring. The first cantilever is fixed relative to, or attached to, the first sled and the first seat is fixed relative to, or attached to, the support structure. Worded differently, the first cantilever may form part of the first sled and the first seat may form part of the support structure. The first cantilever, or a tip of the first cantilever, may be biased, or referenced, against the first seat in the loaded state of the first actuator, or more precisely in the loaded position of the first sled. The trigger may comprise, or form, a first cam element that compresses the first cantilever and releases the first cantilever from the first seat at the first position of the trigger, or at a transition from the rest position to the first position. It is understood that the first cam element may be a linear cam element.

The second actuator may comprise a second spring that biases the second actuator from the loaded state to the unloaded state. It is understood that the biasing of the second spring may be smaller in the unloaded state than in the loaded state. It is further understood that the second spring may bias the second actuator with a spring force that is smaller in the unloaded state than in the loaded state. The spring force may decrease linearly between the loaded state and the unloaded state.

More specifically, the second actuator may comprise a second spring and a second sled, or second hammer, wherein the second sled is configured to be connected to, or attached to, the cannula, or the second sled is connected to, or attached to, the cannula. The second sled has a loaded position, or cocked position, and an unloaded position, or uncocked position, relative to the support structure, and the second spring biases the second sled to transition from the loaded position to the unloaded position. More generally, the second spring biases the biopsy needle to transition from the open configuration to the closed configuration. It is understood that the loaded position is in the loaded state of the second actuator and that the unloaded position is in the unloaded state of the second actuator. It is understood that the second spring may be biased, or referenced, between the second sled and the support structure.

The second sled may be arranged to move, or translate, linearly between the loaded position and the unloaded position. The support structure and/or the trigger may form a second track, or second guide, that restricts the second sled to a linear transition between the loaded position and the unloaded position.

The biopsy gun, or the second actuator, may comprise a second catch arrangement, or second sear mechanism, arranged to releasably lock, or arrest, the second actuator in the loaded state, or more precisely to releasably lock, or arrest, the second sled in the loaded position. The trigger may be arranged to cooperate with the second catch arrangement and to activate the second actuator, to unlock, or release, the second actuator from the loaded state, or more precisely to unlock, or release, the second sled from the loaded position.

The second catch arrangement may comprise a second cantilever and a second seat, or second post. The second cantilever may be a leaf spring. The second cantilever is fixed relative to, or attached to, the second sled and the second seat is fixed relative to, or attached to, the support structure. Worded differently, the second cantilever may form part of the second sled and the second seat may form part of the support structure. The second cantilever, or a tip of the second cantilever, may be biased, or referenced, against the second seat in the loaded state of the second actuator, or more precisely in the loaded position of the second sled. The trigger may comprise, or form, a second cam element that compresses the first cantilever and releases the second cantilever from the first seat at the second position of the trigger, or at a transition from the first position to the second position. It is understood that the second cam element may be a linear cam element.

It is specified that the travel limiter may be arranged to releasably set the second actuator in an intermediate state between the loaded state and the unloaded state of the second actuator. It is further specified that the second actuator may comprise a second sled that has a loaded position and an unloaded position. The second sled may have an intermediate position relative to the support structure. It is understood that the intermediate position is located between the loaded position and the unloaded position of the second sled, and that second sled transitions between the loaded position and the unloaded position via the intermediate position. It is further understood that the intermediate position is in the intermediate state of the second actuator. The cannula may be still, or unmoving, relative to the stylet at the intermediate position of the second sled.

The travel limiter may comprise a third spring and a stop element or block, or retaining element or block, wherein the stop element has a first position and a second position relative to the support structure, and the third spring biases the stop element from the second position to the first position. In the first position, the stop element may set, or arrest, the second actuator in the intermediate state at a transition between the loaded state and the unloaded state. In the second position, the stop element may allow the second actuator to transition between the intermediate state and the unloaded state.

In the first position, the stop element may set, or arrest, the second sled in the intermediate position at a transition between the loaded position and the unloaded position. More specifically, in the intermediate state of the second actuator and with the stop element in the first position, the stop element may prevent the second actuator from transitioning to the unloaded state. Worded differently, in the intermediate position of the second sled and with the stop element in the first position, the stop element may prevent the second sled from transitioning to the unloaded position.

In the second position, the stop element may allow the second actuator to transition between the intermediate position and the unloaded position. More specifically, in the intermediate state of the second actuator and with the stop element in the second position, the stop element may allow the second actuator to transition to the unloaded state. Worded differently, in the intermediate position of the second sled and with the stop element in the second position, the stop element may allow the second sled to transition to the unloaded position.

With the second actuator in the intermediate state, or the second sled in the intermediate position, and the stop element in the first position, the second spring may bias the second sled against the stop element. With the second actuator in the unloaded state, or the second sled in the unloaded position, and the stop element in the second position, the second spring may bias the second sled against the stop element and/or the support structure.

The second sled may form a first mechanical interface and the stop element may form a second mechanical interface. The first mechanical interface and the second mechanical interface may cooperate to set, or arrest, the second sled in the intermediate position at a transition between the loaded position and the unloaded position with the stop element in the first position, and to allow the second actuator to transition between the intermediate position and the unloaded position with the stop element in the second position.

It is understood that with the second sled in the intermediate position and the stop element in the first position, the second sled, or the first mechanical interface, may contact stop element, or the second mechanical interface. More specifically, with the second sled in the intermediate position and the stop element in the first position, the second spring may bias the second sled, or the first mechanical interface, against the stop element, or the second mechanical interface.

With the second sled in the unloaded position and the stop element in the second position, the second sled, or the first mechanical interface, may contact stop element, or the second mechanical interface. More specifically, with the second sled in the unloaded position and the stop element in the second position, the second spring may bias the second sled, or the first mechanical interface, against the stop element, or the second mechanical interface.

With the second sled in the unloaded position and the stop element in the second position, the second sled may prevent the stop element from transitioning to the first position. More specifically, with the second sled in the unloaded position, the first mechanical interface and the second mechanical interface may cooperate and set, or arrest, the stop element in the second position, or cooperate to prevent the stop element from transitioning from the second position to the first position.

With the second sled in the unloaded position and the stop element in the second position, the third spring may bias the stop element, or the second mechanical interface, against the second sled, or the first mechanical interface. It is understood that the third spring may be biased between the stop element and the support structure.

It is specified above that the trigger may have a third position relative to the support structure, and that the trigger may activate, or release, the travel limiter at the third position. The trigger may comprise, or form, a third cam element. More specifically, the trigger, or the third cam element, may shift the stop element from the first position to the second position at the third position of the trigger, or at a transition from the second position to the third position of the trigger. It is understood that the third cam element may be a linear cam element.

The stop element may be arranged to move, or translate, linearly between the first position and the second position. The support structure may form a stop-element track, or stop-element guide, that restricts the stop element to a linear transition between first position and the second position.

It is specified that the second actuator may be a linear actuator, and that it may have a linear action. It is further specified that the second sled may be arranged to move linearly between the loaded position and the unloaded position. The travel limiter, or stop element, may be arranged to move, or translate, transversely, or perpendicularly, to the linear action of the second actuator. Worded differently, the travel limiter, or stop element, may be arranged to transition transversely, or perpendicularly, to the transition between the loaded state and the unloaded state of the second sled.

The biopsy gun may further comprise: a ratchet, or ratchet arrangement. It is understood that the ratchet may be a linear ratchet. The ratchet may be arranged to prevent the trigger from transitioning in a direction from the third position to the first position at a transition between the first position and the third position. It is understood that the ratchet may have a backlash at a transitioning of the trigger between the first position and the third position, and that the backlash is smaller than the distance between the first position and the second position, and smaller than the distance between the second position and the third position. The ratchet may be arranged to release the trigger at the third position and allow the trigger to transition from the third position to the first position, or to the rest position.

More specifically, the ratchet may be arranged to allow the trigger to transition from the first position to the second position, and to prevent the trigger from transitioning from the second position to the first position. The ratchet may be arranged to allow the trigger to transition from the second position to between the second position and the third position, and to prevent the trigger from transitioning from the between second position and the third position to the second position.

The ratchet may comprise a pawl and a gear rack. The pawl is fixed relative to, or attached to, the travel limiter, or stop element, and the gear rack is fixed relative to, or attached to, the trigger. Worded differently, the pawl may form part of the travel limiter, or stop element, and the gear rack may form part of the trigger. The third spring of the travel limiter may bias, or reference, the pawl against the gear rack, for example via the travel limiter, or stop element. The pawl may engage, or cooperate with, gear rack at the first position of the trigger and between the first position and the third position of the trigger. The pawl may engage, or cooperate with, the gear rack to prevent the trigger from transitioning from the second position to the first position, from between the first position and the second position to the first position, and/or from between the second position and the third position to the second position. The pawl may engage, or cooperate with, the gear rack with the stop element in the first position. The pawl may engage, or cooperate with, the gear rack with the travel limiter, or stop element, between the first position and the second position of the stop element. The pawl may be released from, or lifted from, the gear rack at the third position of the trigger, or at the second position of the stop element. At this stage, the return spring of the trigger arrangement may cause the trigger to transition to the first position, or rest position.

The biopsy gun may further comprise: a lock, or safety. The lock may be configured to, or arranged to, prevent the travel limiter from being activated at a transition from the closed configuration to the open configuration of the biopsy needle, and or at a transition between the open configuration and the intermediate configuration of the biopsy needle. The lock may be configured to, or arranged to, allow the travel limiter to be activated at the intermediate configuration of the biopsy needle. More specifically, the lock may be configured to, or arranged to, prevent the travel limiter from being activated, or to prevent the stop element from transitioning from the first position to the second position, with the second actuator in the loaded state, or with the second sled in the loaded position. The lock may be configured to, or arranged to, allow the travel limiter to be activated, or to allow the stop element to transition from the first position to the second position, with the second actuator in the intermediate state, or with the second sled in the intermediate position. The lock may be configured to, or arranged to, cooperate with the second actuator, or the second sled, to allow the travel limiter to be activated, or to allow the stop element to transition from the first position to the second position.

The lock may comprise a lock spring, or a safety spring, and a lock element or block, or a safety element or block. The lock spring may bias the lock element towards the second actuator. The lock element may have a closed position and an open position relative to the support structure, and the lock spring may bias the lock element from the open position towards the closed position. With the second actuator in the loaded state, or the second sled in the loaded position, the lock spring may bias the lock element against the support structure, or against the stop element. It is understood that the lock spring may be biased between, or referenced between, the lock element and the support structure.

In the closed position, the lock element may prevent the travel limiter from being activated, or prevent, or block, the stop element from transitioning from the first position to the second position. In the open position, the lock element may allow the travel limiter to be activated, or allow the stop element to transition from the first position to the second position. More specifically, in the closed position, the lock element may be located within the stop-element track and in the open position, the lock element may be located outside the stop-element track.

In the loaded state, the second actuator may be spaced apart from the lock, or the lock element. More specifically, in the loaded position, the second sled may be spaced apart from the lock, or the lock element. At the intermediate state, the actuator may engage, or contact, the lock, or the lock element. More specifically, in the intermediate position, the second sled may engage, or contact, the lock, or the lock element. The second actuator may bias, or reference, the stop element and transition it from the closed position to the open position at a transition from the loaded state to the intermediate state. More specifically, the second sled may bias, or reference, the stop element and transition it from the closed position to the open position at a transition from the loaded position to the intermediate position.

The lock element may have an end position relative to the support structure. It is understood that the open position is located between the end position and the closed position. At the unloaded state, the actuator may engage, or contact, the lock, or the lock element. More specifically, in the unloaded position, the second sled may engage, or contact, the lock, or the lock element. The second actuator may bias, or reference, the stop element and transition it from the open position to the end position at a transition from the intermediate state to the unloaded state. More specifically, the second sled may bias, or reference, the stop element and transition it from the open position to the end position at a transition from the intermediate position to the unloaded position. In the unloaded state, the second actuator may bias, or reference, the stop element against the support structure. More specifically, in the unloaded position, the second sled may bias, or reference, the stop element against the support structure.

The lock spring of the damper may be arranged to counter the second spring of the second actuator. It is understood that this may be at the unloaded state, at the intermediate state, and/or between the intermediate state and the unloaded state. The lock spring may be aligned with the second spring.

The lock element may be arranged to move, or translate, linearly between the closed position and the open position, or the end position. The support structure may form a lock-element track, or lock-element guide, that restricts the lock element to a linear transition between closed position and the open position, or the end position.

It is specified that the second actuator may be a linear actuator, and that it may have a linear action. It is further specified that the second sled may be arranged to move linearly between the loaded position and the unloaded position. The lock, or lock element, may be arranged to move, or translate, along, or parallel with, the linear action of the second actuator. Worded differently, the lock, or lock element, may be arranged to transition along, or parallel with, the transition between the loaded position and the unloaded position of the second sled.

The lock may be a damper, or counter. It is understood that the lock may be a combined lock and damper. Alternatively, the biopsy gun may further comprise a damper, or counter. It is further understood that the damper may be a linear damper, and that the damper may be configured, or arranged, to allow the biopsy needle to reach the closed configuration, the second actuator to reach the unloaded state, or the second sled to reach the unloaded position.

The damper may be configured to, or arranged to, dampen, or counter, the second actuator at a transition of the biopsy needle to the closed configuration, for example from the open configuration and/or from the intermediate configuration. The damper may be configured to, or arranged to, dampen, or counter, the second actuator with the biopsy needle at the closed configuration, with the second actuator at the unloaded state, or with the second sled at the unloaded position. The damper may be configured to, or arranged to, dampen, or counter, the second actuator with the biopsy needle at the intermediate configuration, or with the second actuator at the intermediate state, or with the second sled at the intermediate position. The damper may be configured to, or arranged to, dampen, or counter, the second actuator with the biopsy needle between the intermediate configuration and the closed configuration, or with the second actuator between the intermediate state and the unloaded state, or with the second sled between the intermediate position and the unloaded position.

The lock spring may be a damper spring, or a combined lock and damper spring. The lock element may be damper element or block, or a combined lock and damper element or block. The damper spring may bias the damper element towards the second actuator. The damper element may have a first position and a second position relative to the support structure, and the damper spring may bias the damper element from the second position towards the first position. The first position of the damper element may correspond to the closed position of the stop element, and the second position of the damper element may correspond to the end position of the stop element. With the second actuator in the loaded state, or the second sled in the loaded position, the damper spring may bias the damper element against the support structure, or against the stop element. It is understood that the damper spring may be biased between, or referenced between, the damper element and the support structure.

In the loaded state, the second actuator may be spaced apart from the damper, or the damper element. More specifically, in the loaded position, the second sled may be spaced apart from the damper, or the damper element. The damper element may engage, or contact, the second actuator, or the second sled, with the biopsy needle at the closed configuration, with the second actuator at the unloaded state, or with the second sled at the unloaded position. The damper element may engage, or contact, the second actuator, or the second sled, with the biopsy needle at the intermediate configuration, with the second actuator at the intermediate state, or with the second sled at the intermediate position. The damper element may engage, or contact, the second actuator, or the second sled, with the biopsy needle between the intermediate configuration and the closed configuration, with the second actuator between the intermediate state and the unloaded state, or with the second sled between the intermediate position and the unloaded position.

The damper spring of the damper may be arranged to counter the second spring of the second actuator. It is understood that this may be at the unloaded state, at the intermediate state, and/or between the intermediate state and the unloaded state. The damper spring may be aligned with the second spring.

The damper element may be arranged to move, or translate, linearly between the first position and the second position. The stop-element track may be a damper-element track, or a combined stop and damper-element track, that restricts the damper element to a linear transition between first position and the second position.

It is specified that the second actuator may be a linear actuator, and that it may have a linear action. It is further specified that the second sled may be arranged to move linearly between the loaded position and the unloaded position. The damper, or damper element, may be arranged to move, or translate, along, or parallel with, the linear action of the second actuator. Worded differently, the damper, or damper element, may be arranged to transition along, or parallel with, the transition between the loaded position and the unloaded position of the second sled.

The biopsy gun may further comprise: an indicator. The indicator may be visual and/or tactile. The indicator may be configured to, or arranged to, indicate that the biopsy needle has transitioned from the open configuration to the closed configuration, that the trigger has transition to, or is at, the third position, that the second actuator has transition to, or is at, the unloaded state, and/or that the second sled has transitioned to, or is at, the unloaded position.

It is understood that the support structure may have, or form a housing. It is further understood that support structure may have an inside and an outside. The first actuator, the second actuator, the travel limiter, the ratchet, and/or the lock may be located within the support structure. The support structure, or the housing, may have an aperture that extends from within the support structure to outside the support structure. The aperture may form part of the indicator. It is understood that the support structure, or the housing, may have, or form, a wall, and that the aperture is in the wall. The aperture may be located at the forward end of the support structure. More specifically, the aperture may be located at the side of the support structure from which the biopsy needle extends, or at the opposite side of the support structure from the push button.

The trigger may have a protrusion. The protrusion and the push button may be located at opposite sides of the trigger. The trigger may form part of the indicator. The protrusion may be configured to, or arranged to, move inside the aperture at a transition from the second position to the third position of the trigger. The protrusion may extend through the aperture at the third position of the trigger. The protrusion may extend from the aperture, the support structure, the housing, or the wall, at the third position of the trigger. Alternatively, the protrusion may be flush with the support structure, the housing, or the wall, at the third position of the trigger. The protrusion may extend from the aperture, the support structure, the housing, or the wall, at the third position of the trigger. The protrusion may be retracted inside, or located within, the support structure, the housing, and/or the aperture, in at the first position and the second position of the trigger.

More specifically, the protrusion may have a tip. The tip may be is located within the support structure, the housing, or the aperture, at the first position and the second position of the trigger. The tip may be located outside, or be accessible and/or visible from outside, the support structure, or the housing, at the third position of the trigger.

If a first element and a second element jointly forms a monolithic structure, or unitary structure, it is understood that the first elements and the second element are not separate parts in the manufacturing. For example, a monolithic element may be of plastic, and it may be formed by injection molding.

For each of the first catch arrangement and the second catch arrangement, the cantilever and the sled may jointly form a monolithic, or unitary structure. Similarly, the seat and the support structure may jointly form a monolithic, or unitary structure. The trigger, including the first cam element, the second cam element, and the third cam element, may be a monolithic, or unitary, structure. For the ratchet, the pawl and the stop element may jointly form a monolithic, or unitary structure. Similarly, the gear rack and the trigger may jointly form a monolithic, or unitary structure.

Each of the support structure, the first sled, the second sled, the stop element, and the lock element may be of plastics. For example, plastics may be, or comprise, polycarbonate, acrylonitrile butadiene styrene, nylon, or acetate. The plastics may be fiber reinforced, for example with a low level of glass fiber.

It is understood that each of the first spring, the second spring, the third spring, the return spring, and the lock spring may be a mechanical spring. The mechanical spring may be a compression coil spring. The mechanical spring may be a linear rate spring, a progressive rate spring, or a variable rate spring. The mechanical spring may be of metal.

Each of the first spring and the second spring may have a spring constant that is greater than 0.6 N/mm, or greater than 1 N/mm. Each of the first spring and the second spring may have a spring constant that is smaller than 3 N/mm, or smaller than 2 N/mm. The first spring may be compressed in the unloaded state of the first actuator, or in the unloaded position of the first sled. Similarly, the second spring may be compressed in the unloaded state of the second actuator, or in the unloaded position of the second sled.

The second spring may generate a force that is greater than 15 N, or greater than 20 N, in the loaded state of the second actuator, or at the loaded position of the second sled. The second spring may generate a force that is greater than 3 N, or greater than 5 N, in the intermediate state of the first actuator, or at the intermediate position of the first sled. The second spring may generate a force that is greater than 1 N, or greater than 2 N, in the unloaded state of the second actuator, or at the unloaded position of the second sled.

The second spring may generate a force that is smaller than 80 N, or smaller than 60 N, in the loaded state of the second actuator, or at the loaded position of the second sled. The second spring may generate a force that is smaller than 17 N, or smaller than 13 N, in the intermediate state of the first actuator, or at the intermediate position of the first sled. The second spring may generate a force that is smaller than 9 N, or smaller than 7 N, in the unloaded state of the second actuator, or at the unloaded position of the second sled.

For the second spring, it is understood that the force generated by in the intermediate state may be smaller than the force generated in the loaded state, and greater than the force generated in the unloaded state. It is further understood that the force generated in the loaded state is greater than the force generated in the unloaded state.

The lock spring may have a spring constant that is greater than 0.2 N/mm, or greater than 0.3 N/mm. The lock spring may have a spring constant that is smaller than 0.6 N/mm, or smaller than 0.5 N/mm. The lock spring may have a spring constant that is greater than 5 %, or greater than 10 %, of the spring constant of the second spring. The lock spring may have a spring constant that is smaller than 65 %, or smaller than 55 %, of the spring constant of the second spring. It is understood that the spring constant of the lock spring may be smaller than the spring constant of the second spring.

The lock spring may generate a force that is greater than 0.4 N, or greater than 0.6 N, at the closed position of the lock element. The lock spring may generate a force that is greater than 1.0 N, or greater than 1.2 N, at the open position of the lock element. The lock spring may generate a force that is greater than 1.4 N, or greater than 1.6 N, at the end position of the lock element. The lock spring may generate a force that is smaller than 1.2 N, or smaller than 1.0 N, at the closed position of the lock element. The lock spring may generate a force that is smaller than 2.7 N, or smaller than 2.3 N, at the closed position of the lock element. The lock spring may generate a force that is smaller than 3.5 N, or smaller than 3.0 N, at the end position of the lock element. It is understood that the force generated by the lock spring at the closed position and the open position of the lock element may be smaller than the force generated by the second spring at intermediate position of the second sled. It is further understood that the force generated by the lock spring at the end position of the lock element may be smaller than the force generated by the second spring at the unloaded position of the second sled.

It is specified that the biopsy needle comprises a stylet and a cannula, that the biopsy needle has a closed configuration and an open configuration, and that the stylet and the cannula may move relative to one another at a transition between the closed configuration and the open configuration.

It is understood that the cannula may form, or be, a tube. It is understood that the cannula may be elongated, and that it may have a proximal opening and a distal opening. The stylet may comprise a head, or needle tip. It may further comprise a shaft, or needle shaft, wherein the head is attached to, or connected to, the shaft, and the shaft extends through the cannula.

The cannula may impact the head in the closed configuration at a transition from the open configuration, or form the intermediate configuration. The head may arrest the cannula in the closed configuration at a transition from the open configuration, or the intermediate configuration. Worded differently, it may prevent the cannula from moving forward relative to the stylet in the closed configuration, or the head may prevent the cannula from moving past the head of the stylet in the closed configuration, or at a transition to the closed configuration.

The head may have a shape, or form, and/or size that prevents it from entering into the cannula. More specifically, the cannula may have an internal, or inner, transverse extension, or diameter. The head may have an external, or outer, transverse extension, or diameter, that is greater than the internal transverse extension of the cannula. The transverse extension is understood to be relative to the cannula. This means that the head is prevented from entering the cannula.

The head may engage, or contact, the cannula, in the closed configuration of the biopsy needle. More specifically, the head may have a closed position relative to the cannula in which the head engages, or contacts, the cannula at the distal opening. Worded differently, the head may be located at the distal opening in the closed position of the head.

The head may be disengaged from, or spaced apart from, the cannula, in the open configuration of the biopsy needle. More specifically, the head may have open position relative to the cannula in which the head is spaced apart from the cannula, or the distal opening of the cannula. Worded differently, the head may be spaced apart from the distal opening in the open position of the head.

It is understood that the shaft extends from the cannula at the proximal opening, both in the closed position and in the open position of the head, or in the closed configuration and the open configuration of the biopsy needle. It is further understood that the shaft extends along and within, or through, the cannula.

It is understood that the cannula may be biased against the head of the stylet, for example by the second spring, in the unloaded state of the second actuator, or in the unloaded position of the second sled. It is further understood that the biasing may be in a direction along, or parallel to, the biopsy needle, or more specifically along the cannula.

The cannula and the head may jointly form an outer surface, or external surface, at the distal opening in the closed position of the head, or in the closed configuration of the biopsy needle. The cannula and the head may form an intersection between them in the outer surface. The outer surface may have a cylindrical geometry, such as a circular-cylindrical geometry. The head may seal to the cannula in the closed position.

The cannula and the stylet, or more precisely the cannula and the shaft of the stylet, may jointly form a cavity with the head in the closed position, or in the closed configuration of the biopsy needle, for holding a biopsy sample. It is understood that the cavity may be covered, or enclosed, by the cannula. For example, the shaft may have, or form, a cutout, or holding space, that together with the cannula form a cavity in the closed configuration of the biopsy needle that can hold a biopsy sample. It is understood that the cutout may be covered, or enclosed, by the cannula in the closed configuration. The cutout may be exposed, or fully exposed, in the open configuration. It is understood that the cutout may be located outside the cannula in the open configuration.

The head may form a tip or cutting edge for penetrating tissue. The cannula may form a cutting edge for cutting a biopsy sample from tissue. The cutting edge of the cannula may be formed at, or by, the distal opening of the cannula. The cutting edge of the cannula may pass over the abovementioned cavity, or cutout, at a transition from the open configuration to the closed configuration of the biopsy needle.

It is specified that the first actuator is configured to be coupled, or is coupled to the stylet. More specifically, the first actuator may be configured to be coupled to the shaft of the stylet, or is coupled to the shaft of the stylet. It is further specified that the first sled is configured to be connected to the stylet, or the first sled is connected to the stylet. More specifically, the first sled may be configured to be connected to the shaft of the stylet, or the first sled is connected to the shaft of the stylet.

### Description of the drawings

Figures 1a and 1b are a perspective views of an embodiment of a biopsy arrangement composed of a biopsy needle and a biopsy gun.
Figures 2a and 2b are exploded perspective views of the biopsy arrangement.
Figures 3a, 3b, 3c, and 3d are side views of the front end of the biopsy needle in the closed configuration, the open configuration, the intermediate configuration, respective the closed configuration.
Figure 4a is a cross-sectional view of the biopsy gun with the first actuator in the loaded state, the second actuator in the loaded state, and the trigger between the rest position and the first position.
Figure 4b is a cross-sectional view of the biopsy gun with the first actuator in the loaded state, the second actuator in the loaded state, and the trigger in the first position.
Figure 4c is a cross-sectional view of the biopsy gun with the first actuator in the unloaded state, the second actuator in the loaded state, and the trigger between the first position and the second position.
Figure 4d is a cross-sectional view of the biopsy gun with the first actuator in the unloaded state, the second actuator in the loaded state, and the trigger in the second position.
Figure 4e is a cross-sectional view of the biopsy gun with the first actuator in the unloaded state, the second actuator in the intermediate state, the trigger between the second position and the third position, and the stop element in the first position.
Figure 4f is a cross-sectional view of the biopsy gun with the first actuator in the unloaded state, the second actuator in the intermediate state, the trigger in the third position, and the stop element in the second position.
Figure 4g is a cross-sectional view of the biopsy gun with the first actuator in the unloaded state, the second actuator in the unloaded state, the trigger between the third position and the rest position, and the stop element in the second position.

### Detailed description

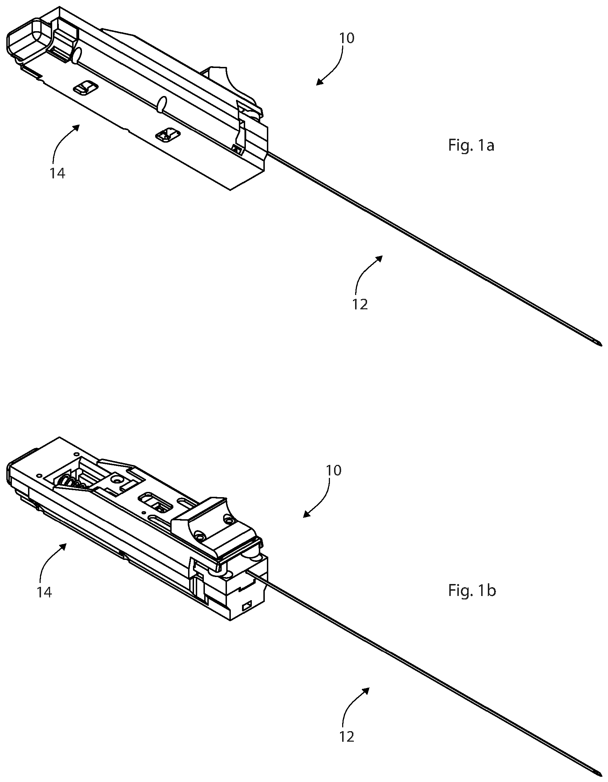

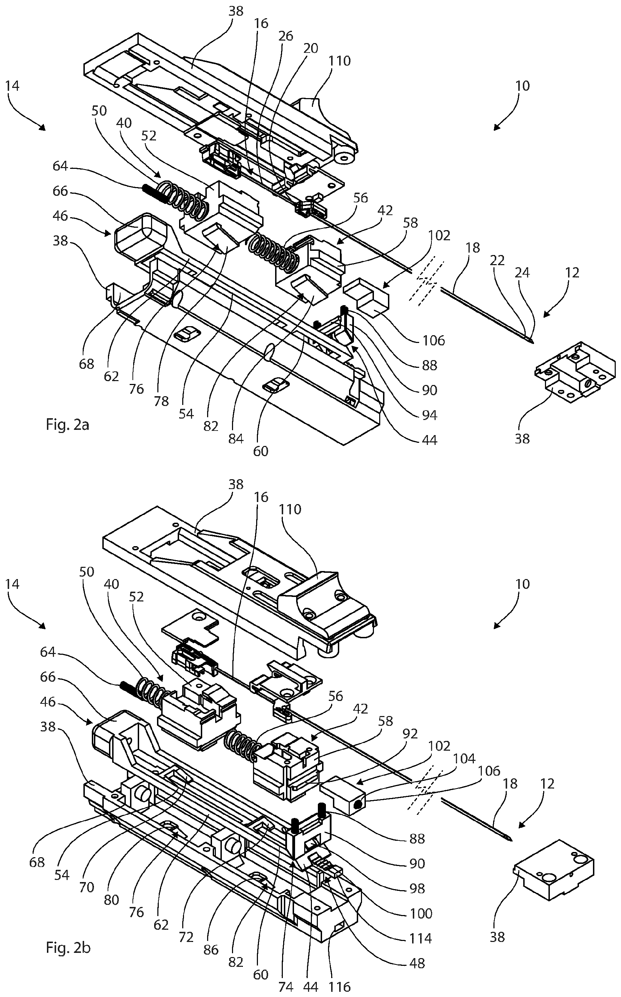

An embodiment of a biopsy arrangement 10 is shown in Figures 1 to 4.

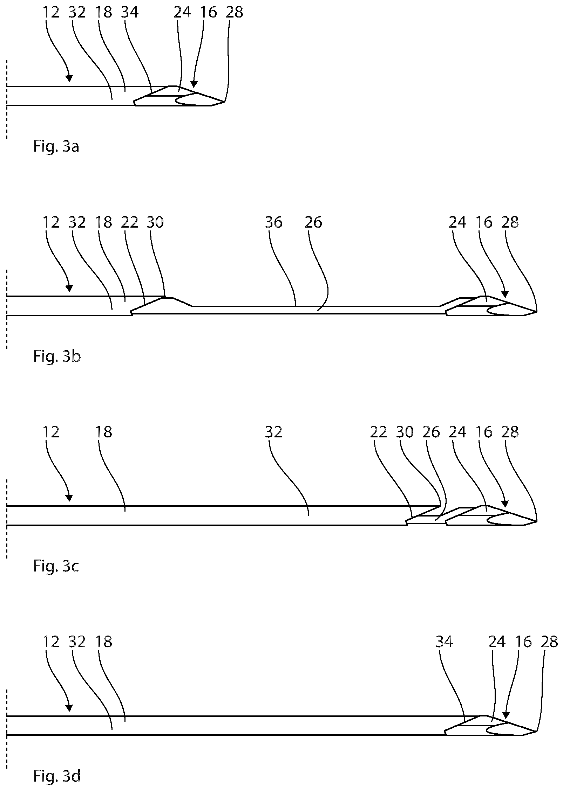

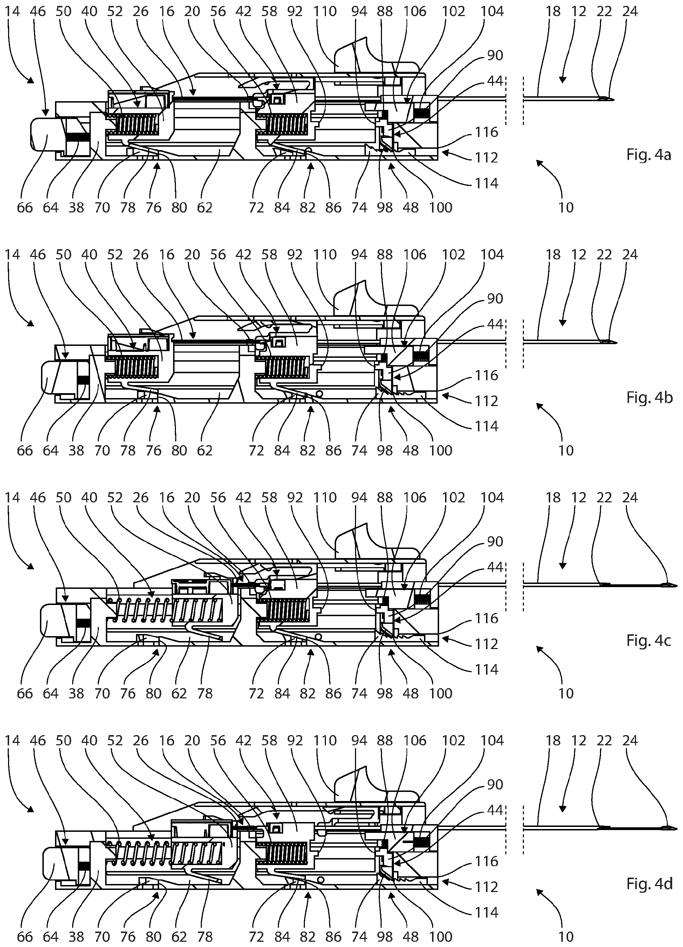

With reference to Figures 3a to 3d, the biopsy arrangement 10 has a biopsy needle 12 with a stylet 16 a cannula 18. The cannula 18 forms an elongated tube and has a proximal opening 20 and a distal opening 22, see Figures 4a to 4g. The stylet 16 has a head 24 and a shaft 26. The head 24 is attached to the shaft 26, and the shaft 26 extends through the cannula 18. The head 24 forms a cutting edge 28 for penetrating tissue. The cannula 18 forms a cutting edge 30 for cutting a biopsy sample from tissue.

The biopsy needle 12 can transition from an initial closed configuration to an open configuration, from the open configuration to an intermediate configuration, and from the intermediate configuration to a final closed configuration. The closed configurations are shown in Figures 3a and 3d, the open configuration is shown in Figure 3b, and the intermediate configuration is shown in Figure 3c. The stylet 16 and the cannula 18 move relative to one another at a transition between the configurations. The head 24 contacts the cannula 18 at the distal opening 22 in the closed configurations. The head 24 is spaced apart from the cannula 18 and the distal opening 22 in the open configuration and the intermediate configuration. The shaft 26 extends from the cannula 18 at the proximal opening 20, both in the closed configuration and in the open configuration of the biopsy needle 12.

The cannula 18 and the head 24 jointly forms an outer surface 32 with a circular-cylindrical geometry at the distal opening 22 in the closed configuration of the biopsy needle 12. The cannula 18 and the head 24 forms an intersection 34 in the outer surface 32, and the head 24 seals to the cannula 18. The cannula 18 has an inner diameter, and the head 24 has an outer diameter that is greater than the inner diameter of the cannula 18. This way, the head 24 has a shape that prevents it from entering the cannula 18. This way, at a transition from the open configuration of the biopsy needle 12, the cannula 18 impacts the stylet 16 in the closed configuration and the stylet 16 stops the cannula 18. The stylet 16 also prevents the cannula 18 from moving forward relative to the stylet 16 in the closed configuration.

The shaft 26 has a cutout 36 that together with the cannula 18 form a cavity that can hold a biopsy sample in the closed configuration of the biopsy needle 12. The cavity is covered by the cannula 18 in the closed configuration. The cutout 36 is fully exposed and located outside the cannula 18 in the open configuration, and the cutting edge of the cannula 18 passes over the cutout 36 at a transition from the open configuration to the closed configuration of the biopsy needle 12.

The biopsy arrangement 10 further has a biopsy gun 14. The biopsy gun 14 has a support structure 38, a first actuator 40, a second actuator 42, a travel limiter 44, a trigger arrangement 46, and a ratchet 48. The biopsy needle 12 extends from the support structure 38. The support structure 38 is elongated and the biopsy needle 12 is aligned with the elongated support structure 38, as shown in Figures 1a and 1b.

The first actuator 40 is a linear actuator that has a first spring 50 and a first sled 52. The first spring 50 is biased between the first sled 52 and the support structure 38. The first sled 52 is attached to the shaft 26 of the stylet 16. The first sled 52 has a loaded position and an unloaded position relative to the support structure 38. The support structure 38 and the trigger 62 form a first track 54 that restricts the first sled 52 to a linear transition between the loaded position and the unloaded position. The first spring 50 biases the first sled 52 to transition from the loaded position to the unloaded position, and in extension the biopsy needle 12 to transition from the closed configuration to the open configuration.

With the first sled 52 in the loaded position, the first actuator 40 is in a loaded state, and with the first sled 52 in the unloaded position, the first actuator 40 is in an unloaded state. In Figures 4a and 4b, the first actuator 40 is in the loaded state and the first sled 52 is in the loaded position, and in Figures 4c to 4g the first actuator 40 is in the unloaded state and the first sled 52 is in the unloaded position.

The second actuator 42 is a linear actuator that has a second spring 56 and a second sled 58. The second spring 56 is biased between the second sled 58 and the support structure 38. The second sled 58 is attached cannula 18. The second sled 58 has a loaded position, an intermediate position, and an unloaded position relative to the support structure 38. The intermediate position is located between the loaded position and the unloaded position of the second sled 58. The support structure 38 and the trigger 62 form a second track 60 that restricts the second sled 58 to a linear transition between the loaded position and the unloaded position. The second spring 56 biases the second sled 58 to transition from the loaded position to the unloaded position, and in extension the biopsy needle 12 to transition from the closed configuration to the open configuration.

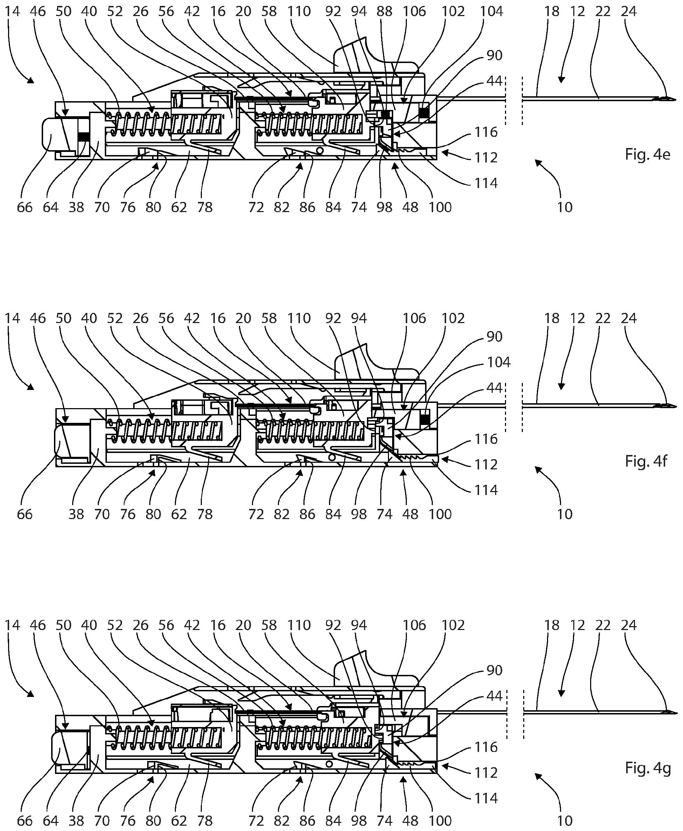

With the second sled 58 in the loaded position, the second actuator 42 is in a loaded state, with the second sled 58 in the intermediate position, the second actuator 42 is in the intermediate state, and with the second sled 58 in the unloaded position, the second actuator 42 is in an unloaded state. In Figures 4a to 4d the second actuator 42 is in the loaded state and the second sled 58 is in the loaded position, in Figures 4e and 4f the second actuator 42 is in the intermediate state and the second sled 58 is in the intermediate position, and in Figure 4g the second actuator 42 is in the unloaded loaded state and the second sled 58 is in the unloaded position.

Arranged this way, the first actuator 40 and the second actuator 42 are configured to set the biopsy needle 12 in the closed configuration with the first actuator 40 in the loaded state and the second actuator 42 in the loaded state, to set the biopsy needle 12 in the open configuration with the first actuator 40 in the unloaded state and the second actuator 42 in the loaded state, to set the biopsy needle 12 in the intermediate configuration with the first actuator 40 in the unloaded state and the second actuator 42 in the intermediate state, and to set the biopsy needle 12 in the closed configuration with the first actuator 40 in the unloaded state and the second actuator 42 in the unloaded state.

The trigger arrangement 46 has a trigger 62 that is a rigid structure. The trigger 62 has a rest position, a first position, a second position, and a third position, relative to the support structure 38. The trigger 62 is between the rest position and the first position in Figure 4a, in the first position in Figure 4b, between the first position and the second position in Figure 4c, in the second position in Figure 4d, between the second position and the third position in Figure 4e, in the third position in Figure 4f, and between the third position and the rest position in Figure 4g.

The trigger 62 transitions between the first position and the third position via the second position, and between the rest position and the third position via the first position and the second position. The trigger arrangement 46 has a return spring 64 that is biased between the trigger 62 and the support structure 38 and pushes the trigger 62 towards the rest position.

The trigger 62 forms a push button 66 that is accessible from outside the support structure 38. The trigger 62 is manually operated and transitions between the different positions by pushing the push button 66 relative to the support structure 38. The support structure 38 forms a trigger track 68 that restricts the trigger 62 to a linear transition between the rest position, the first position, the second position, and the third position. This way, the trigger 62 is arranged to translate linearly between the different positions.

The biopsy gun 14 has a first catch arrangement 76 that can releasably lock the first actuator 40 in the loaded state. More precisely, the first catch arrangement 76 can releasably lock the first sled 52 in the loaded position. The first catch arrangement 76 has a first cantilever 78 in the form of a leaf spring that is fixed relative to and forms part of the first sled 52. It further has a first seat 80 that is fixed relative to and forms part of the support structure 38. The first cantilever 78 has a tip that is biased against the first seat 80 in the loaded state of the first actuator 40, or more precisely in the loaded position of the first sled 52. The trigger 62 forms a linear first cam element 70 that compresses the first cantilever 78 and releases the first cantilever 78 from the first seat 80 at the first position of the trigger 62. This way, the trigger 62 is arranged to cooperate with the first catch arrangement 76 and to release the first actuator 40 from the loaded state, and in extension to activate the first actuator 40.

Similarly, the biopsy gun 14 has a second catch arrangement 82 that can releasably lock the second actuator 42 in the loaded state. More precisely, the second catch arrangement 82 can releasably lock the second sled 58 in the loaded position. The second catch arrangement 82 has a second cantilever 84 in the form of a leaf spring that is fixed relative to and forms part of the second sled 58. It further has a second seat 86 that is fixed relative to and forms part of the support structure 38. The second cantilever 84 has a tip that is biased against the second seat 86 in the loaded state of the second actuator 42, or more precisely in the loaded position of the second sled 58. The trigger 62 forms a linear second cam element 72 that compresses the second cantilever 84 and releases the second cantilever 84 from the second seat 86 at the second position of the trigger 62. This way, the trigger 62 is arranged to cooperate with the second catch arrangement 82 and to release the second actuator 42 from the loaded state, and in extension to activate the second actuator 42.

The travel limiter 44 has a third spring 88 and a stop element 90. The stop element 90 has a first position and a second position relative to the support structure 38. The third spring 88 is biased between the stop element 90 and the support structure 38, and it biases the stop element 90 from the second position to the first position.

The second sled 58 forms a first mechanical interface 92 and the stop element 90 forms a second mechanical interface 94. The second spring 56 biases the first mechanical interface 92 against the second mechanical interface 94 in the intermediate position of the second sled 58. The first mechanical interface 92 and the second mechanical interface 94 allow the second sled 58 to transition between the intermediate position and the unloaded position with the stop element 90 in the second position.

This way, in the first position, the stop element 90 sets the second sled 58 in the intermediate position at a transition between the loaded position and the unloaded position, and in the second position, the stop element 90 allows the second sled 58 to transition between the intermediate position and the unloaded position. In extension, in the first position, the stop element 90 sets the second actuator 42 in the intermediate state at a transition between the loaded state and the unloaded state, and in the second position, the stop element 90 allows the second actuator 42 to transition between the intermediate state and the unloaded state.

The support structure 38 forms a buffer-stop track (not indicated) that restricts the stop element 90 to a linear transition between first position and the second position. This way, the stop element 90 is arranged to move linearly between the first position and the second position. The linear transition of the stop element 90 is transverse to the linear action of the second actuator 42.

The trigger 62 forms a linear third cam element 74 that shifts the stop element 90 from the first position to the second position at a transition from the second position to the third position of the trigger 62. This way, the trigger 62 activates and releases the travel limiter 44 at the third position.

When the second sled 58 is in the unloaded position and the stop element 90 is in the second position, the third spring 88 biases the second mechanical interface 94 against the first mechanical interface 92, and the first mechanical interface 92 and the second mechanical interface 94 cooperate and prevents the stop element 90 from transitioning from the second position to the first position.

Arranged as described above, the travel limiter 44 releasably set the second actuator 42 in an intermediate state between the loaded state and the unloaded state of the second actuator 42. In the intermediate state, the second actuator 42 and the travel limiter 44 jointly set the biopsy needle 12 in the intermediate configuration. The travel limiter 44 is configured to allow the second actuator 42 to transition from the intermediate state to the unloaded state at an activation of the travel limiter 44, and in extension to allow the second actuator 42 to transition the biopsy needle 12 from the intermediate configuration to the closed configuration at an activation of the travel limiter 44.

The first actuator 40 has a first stroke length between the loaded state and an unloaded state, and the second actuator 42 has a second stroke length between the loaded state and the unloaded state of the second actuator 42 that is the same as the first stroke length. The second actuator 42 further has an intermediate stroke length between the intermediate state and the unloaded state, and the intermediate stroke length is about 12% of the second stroke length.

The biopsy gun 14 further has a linear ratchet 48. The ratchet 48 has a pawl 98 that is fixed relative and forms part of to the stop element 90. It further has a gear rack 100 that is fixed relative to and forms part of the trigger 62. The third spring 88 of the travel limiter 44 biases the pawl 98 against the gear rack 100 such that the pawl 98 engages the gear rack 100.

The pawl 98 engages and cooperates with the gear rack 100 at the first position of the trigger 62 and between the first position and the third position of the trigger 62. This way, the ratchet 48 is arranged to prevent the trigger 62 from transitioning in a direction from the third position to the first position at a transition between the first position and the third position. The pawl 98 engages the gear rack 100 with the stop element 90 in the first position and between the first position and the second position of the stop element 90. The pawl 98 is released from the gear rack 100 at the second position of the stop element 90, which is when the trigger 62 is on the third position. At this stage, the return spring 64 of the trigger arrangement 46 causes the trigger 62 to transition to the rest position. This way, the ratchet 48 is arranged to release the trigger 62 at the third position and allow the trigger 62 to transition from the third position to the rest position.

The biopsy gun further has a lock 102 in the form of a lock spring 104 and a lock element 106, and the lock spring 104 is referenced between the lock element 106 and the support structure 38. The lock spring 104 is a compression coil spring that is aligned parallel with the second spring 56.

The lock element 106 has a closed position and an open position relative to the support structure 38, and the lock spring 104 biases the lock element 106 from the open position to the closed position in a direction towards the second actuator 42. The closed position is shown in Figures 4a to 4d, and the open position is shown in Figures 4e and 4f. With the second actuator 42 in the loaded state, the lock spring 104 biases the lock element 106 against the stop element 90. The support structure 38 forms a lock-element track (not indicated) that restricts the lock element 106 to a linear transition between closed position and the open position, and the lock element 106 is arranged to translate parallel with the linear action of the second actuator 42.

In the closed position, the lock element 106 is located within the stop-element track (not indicated) of the travel limiter 44. In the open position, the lock element 106 is located outside the stop-element track (not indicated). This way, in the closed position, the lock element 106 blocks and prevents the stop element 90 from transitioning from the first position to the second position, and in extensions it prevents the travel limiter 44 from being activated. In the open position, the lock element 106 allows the stop element 90 to transition from the first position to the second position, and in extensions it allows the travel limiter 44 to be activated.

In the loaded state, the second sled 58 is spaced apart from the lock element 106. The second sled 58 engages and biases the lock element 106 at a transition from the loaded position to the intermediate position and transitions the lock element 106 from the closed position to the open position. When the trigger 62 has transitioned the stop element 90 from the first position to the second position, as shown in Figure 4f, the second sled 58 transitions to the unloaded position. At the same time, it biases the lock element 90 to transition from the open position to an end position, which is shown in Figure 4g, at which the second sled 58 references the stop element 90 against the support structure 38.

The lock 102 also constitutes a damper. The lock spring 104 counters the second spring 56 at the intermediate position, between the intermediate position and the unloaded position, and at the unloaded position of the second sled 58. The lock 102 allows the second sled 58 to reach the unloaded position, at which the lock element 106 is at the end position. This way, the lock 102 is configured to dampen and counter the second actuator 42 at the intermediate configuration, between the intermediate configuration and the closed configuration, and at the closed configuration of the biopsy needle.

The biopsy gun 14 further has a charging handle 110 that can be manually racked relative to the support structure 38. The charging handle 110 is operationally coupled to the first sled 52 and the second sled 58. At a first racking, the second sled 58 is transitioned from the unloaded state to the loaded state. This allows for the biopsy sample to me removed from the cutout 36 of the shaft 26. At a second racking, the first sled 52 is transitioned from the unloaded state to the loaded state. This allows for the biopsy arrangement 10 to be used again.

The support structure 38 forms a housing, and the first actuator 40, the second actuator 42, the travel limiter 44, the ratchet 48, and the lock 102 are located within the support structure 38. The support structure 38 has an aperture 116 at the forward end that extends from within the support structure 38 to outside the support structure 38. The trigger 62 has a protrusion 114 that can move inside the aperture 116 at a transition from the second position to the third position of the trigger 62. The protrusion 114 has a tip that is located within the support structure at the first position and the second position of the trigger, as shown in Figures 4b and 4d. The tip is located outside the support structure 38 at the third position of the trigger 62, as shown in Figure 4f, which means that it extends through the aperture 116 and is accessible and visible from outside the support structure 38. This way, the aperture 116 and the protrusion 114 forms a visual and tactile indicator 112 that indicates that the biopsy needle 12 has transitioned from the open configuration to the closed configuration, or more specifically that the second actuator 42 has transitioned to the unloaded state.

Arranged as described above, the support structure 38 operationally support the first actuator 40, the second actuator 42, and the travel limiter 44. Further, with the first actuator 40 in the loaded state and the second actuator 42 in the loaded state, the trigger 62 activates the first actuator 40 at the first position and the second actuator 42 at the second position. The trigger 62 also activates and releases the travel limiter 44 at the third position.

Arranged as described above, the first actuator 40 is configured to transition the biopsy needle 12 from the closed configuration to the open configuration at an activation of the first actuator 40. The first actuator 40 is also coupled to the stylet 16. Further, the second actuator 42 is configured to transition the biopsy needle 12 from the open configuration to the closed configuration at an activation of the second actuator 42. The second actuator 42 is also coupled to the cannula 18.

Arranged as described above, the travel limiter 44 is configured to allow the second actuator 42 to transition the biopsy needle 12 from the intermediate configuration to the closed configuration at an activation of the travel limiter 44. The travel limiter 44 is further arranged to releasably set the biopsy needle 12 in the intermediate configuration between the open configuration and the closed configuration. The travel limiter 44 is further arranged to releasably restrict the second actuator 42 to transition the biopsy needle 12 to the closed configuration at a transition from the open configuration to the closed configuration.

Arranged as described above, the trigger arrangement 46 is operationally connected to the first actuator 40, the second actuator 42, and the travel limiter 44, and the trigger arrangement 46 is configured to sequentially activate the first actuator 40, the second actuator 42, and the travel limiter 44.

### Item list

- 10: biopsy arrangement
- 12: biopsy needle
- 14: biopsy gun
- 16: stylet
- 18: cannula
- 20: proximal opening
- 22: distal opening
- 24: head
- 26: shaft
- 28: cutting edge of head
- 30: cutting edge of cannula
- 32: outer surface
- 34: intersection
- 36: cutout
- 38: support structure
- 40: first actuator
- 42: second actuator
- 44: travel limiter
- 46: trigger arrangement
- 48: ratchet
- 50: first spring
- 52: first sled
- 54: first track
- 56: second spring
- 58: second sled
- 60: second track
- 62: trigger
- 64: return spring
- 66: push button
- 68: trigger track
- 70: first cam element
- 72: second cam element
- 74: third cam element
- 76: first catch arrangement
- 78: first cantilever
- 80: first seat
- 82: second catch arrangement
- 84: second cantilever
- 86: second seat
- 88: third spring
- 90: stop element
- 92: first mechanical interface
- 94: second mechanical interface
- 96: stop-element track
- 98: pawl
- 100: gear rack
- 102: lock
- 104: lock spring
- 106: lock element
- 108: lock-element track
- 110: charging handle
- 112: indicator
- 114: protrusion
- 116: aperture

## Claims

1. A biopsy arrangement (10) comprising a biopsy needle (12) and a biopsy gun (14), wherein
the biopsy needle (12) comprises:
- a stylet (16), and
- a cannula (18), and
the biopsy needle (12) has a closed configuration and an open configuration, wherein
the biopsy gun (14) comprises:
- a first actuator (40),
- a second actuator (42), and
- a travel limiter (44), wherein
the first actuator (40) is coupled to the stylet (16), the first actuator (40) is arranged to transition the biopsy needle (12) from the closed configuration to the open configuration, the second actuator (42) is coupled to the cannula (18), the second actuator (42) is arranged to transition the biopsy needle (12) from the open configuration to the closed configuration, and the travel limiter (44) is arranged to releasably restrict the second actuator (42) to transition the biopsy needle (12) to the closed configuration at a transition from the open configuration to the closed configuration.

2. The biopsy arrangement (10) according to claim 1, wherein the biopsy needle (12) has an intermediate configuration between the open configuration and the closed configuration, and the travel limiter (44) is arranged to releasably set the biopsy needle (12) in an intermediate configuration at the transition from the open configuration to the closed configuration.

3. The biopsy arrangement (10) according to claim 1 or 2, wherein the first actuator (40) is configured to transition the biopsy needle (12) from the closed configuration to the open configuration at an activation of the first actuator (40), the second actuator (42) is configured to transition the biopsy needle (12) from the open configuration to the closed configuration at an activation of the second actuator (42), and the travel limiter (44) is configured to allow the second actuator (42) to transition the biopsy needle (12) from the intermediate configuration to the closed configuration at an activation of the travel limiter (44).

4. The biopsy arrangement (10) according to claim 3, wherein the biopsy gun (14) further comprises:
- a trigger arrangement (46), wherein
the trigger arrangement (46) or is operationally connected to the first actuator (40), the second actuator (42), and the travel limiter (44) wherein the trigger arrangement (46) is configured to sequentially activate the first actuator (40), the second actuator (42), and the travel limiter (44).

5. The biopsy arrangement (10) according to claim 4, wherein the first actuator (40) has a loaded state and an unloaded state, the second actuator (42) has loaded state and an unloaded state, the first actuator (40) and the second actuator (42) are configured to set the biopsy needle (12) in the closed configuration with the first actuator (40) in the loaded state and the second actuator (42) in the loaded state, to set the biopsy needle (12) in the open configuration with the first actuator (40) in the unloaded state and the second actuator (42) in the loaded state, and to set the biopsy needle (12) in the closed configuration with the first actuator (40) in the unloaded state and the second actuator (42) in the unloaded state, and the travel limiter (44) is arranged to releasably set the second actuator (42) in an intermediate state between the loaded state and the unloaded state.

6. The biopsy arrangement (10) according to claim 5, wherein the second actuator (42) comprises a second spring (56) that biases the second actuator (42) from the loaded state to the unloaded state, and that the biasing of the second spring (56) is smaller in the unloaded state than in the loaded state.

7. The biopsy arrangement (10) according to claim 5 or 6, wherein the biopsy gun (14) comprises:
- a support structure (38), wherein
the trigger arrangement (46) comprises a trigger (62), the trigger (62) has a first position, a second position, and a third position relative to the support structure (38), the trigger (62) activates the first actuator (40) at the first position, the trigger (62) activates the second actuator (42) at the second position, and the trigger (62) activates the travel limiter (44) at the third position.

8. The biopsy arrangement (10) according to claim 7, wherein the travel limiter (44) comprises a third spring (88) and a stop element (90), the stop element (90) has a first position and a second position relative to the support structure (38), the third spring (88) biases the stop element (90) from the second position to the first position, in the first position, the stop element (90) sets the second actuator (42) in the intermediate state at a transition between the loaded state and the unloaded state, and in the second position, the stop element (90) allows the second actuator (42) to transition between the intermediate state and the unloaded state.

9. The biopsy arrangement (10) according to claim 8, wherein the trigger (62) comprises a third cam element (74) that shifts the stop element (90) from the first position to the second position at a transition from the second position to the third position of the trigger (62).

10. The biopsy arrangement (10) according to any of the claims 7 to 9, wherein the biopsy gun (14) further comprise:
- a ratchet (48), wherein
the ratchet (48) is arranged to prevent the trigger (62) from transitioning in a direction from the third position to the first position at a transition between the first position and the third position.

11. The biopsy arrangement (10) according to claim 10, wherein the ratchet (48) is be arranged to release the trigger (62) at the third position and allow the trigger (62) to transition from the third position to the first position.

12. The biopsy arrangement (10) according to claim 11, wherein the trigger arrangement (46) comprises a return spring arranged to bias the trigger (62) towards the first position.

13. The biopsy arrangement (10) according to any of the claims 5 to 12, wherein the second actuator (42) has a second stroke length between the loaded state and the unloaded state, the second actuator (42) has an intermediate stroke length between the intermediate state and the unloaded state, and the intermediate stroke length is greater than 2%, 5%, or 10%, of the second stroke length.

14. The biopsy arrangement (10) according to claim 13, wherein the intermediate stroke length is smaller than 20%, or 15%, of the second stroke length.

15. The biopsy arrangement (10) according to any of the claims 1-14, wherein stylet (16) comprises a head (24) and a shaft (26), wherein the head (24) is attached to the shaft (26), the shaft (26) extends through the cannula (18), the first actuator (40) is coupled to the shaft (26), the head (24) has a shape and/or size that prevents it from entering into the cannula (18), and the head (24) engages the cannula (18) in the closed configuration of the biopsy needle (12).
